# EUROPEAN PATENT SPECIFICATION

(11) **EP 3 464 603 B1**
(45) Date of publication and mention of the grant of the patent: **26.02.2020**
(21) Application number: 17726611.1
(22) Date of filing: 24.05.2017
(51) Int. Cl.: C12P 7/04, C12P 7/52

(54) **BIOTECHNOLOGICAL PRODUCTION OF PROPANOL AND/OR PROPIONIC ACID IN THE PRESENCE OF ACETATE**
BIOTECHNOLOGISCHE HERSTELLUNG VON PROPANOL UND/ODER PROPIONSÄURE IN GEGENWART VON ACETAT
PRODUCTION BIOTECHNOLOGIQUE DE PROPANOL ET/OU D'ACIDE PROPIONIQUE EN PRÉSENCE D'ACÉTATE

(30) Priority: 27.05.2016 EP 16171624
(43) Date of publication of application: 10.04.2019
(73) Proprietor: Evonik Operations GmbH, 45128 Essen (DE)
(72) Inventor: HAAS, Thomas, 48161 Münster (DE); BECK, Simon, 48161 Münster (DE); HECKER, Anja, 48147 Münster (DE)
(74) Representative: Evonik Patent Association
(86) International application number: PCT/EP2017/062642
(87) International publication number: WO 2017/202975

(56) References cited:
- WO-A1-2014/140336
- CHRIS C. STOWERS ET AL: "Development of an industrializable fermentation process for propionic acid production", JOURNAL OF INDUSTRIAL MICROBIOLOGY AND BIOTECHNOLOGY, vol. 41, 2014, pages 837-852, XP035330832,
- MAKOTO ATO ET AL: "Enrichment of amino acid-oxidizing, acetate-reducing bacteria", JOURNAL OF BIOSCIENCE AND BIOENGINEERING, vol. 118, 2014, pages 160-165, XP002762100,
- J.L. THOLOZAN ET AL: "Clostridium neopropionicum sp. nov., a strict anaerobic bacterium fermenting ethanol to propionate through acrylate pathway", ARCHIVES OF MICROBIOLOGY, vol. 157, 1992, pages 249-257, XP002762101,

## Description

### FIELD OF THE INVENTION

The present invention relates to a biotechnological method for producing propanol and/or propionic acid. In particular, acetate is maintained at a concentration of at least 1 g/L in the aqueous medium during the reaction period.

### BACKGROUND OF THE INVENTION

Propanol is a solvent used in the pharmaceutical industry for resins and cellulose esters amongst other compounds. This solvent, which is better known as isopropanol or isopropyl alcohol, is widely used on printing ink and in the printing industry. 1-propanol is produced in nature by the decomposition of organic materials by a variety of microorganisms and may be found in plants and fusel oil. 1-propanol can also be produced from petrochemically-derived ethene by a reaction with carbon monoxide and hydrogen to give propionaldehyde, which is then hydrogenated. It is also a byproduct of methanol manufacture and may be produced from propane directly or from acrolein.

Propanol has other potential uses. One of the important uses of propanol is that it can be readily dehydrated to produce propylene which is one of the largest chemical commodities in the world. For example, isopropanol (IPA) is currently produced using propylene. In particular, by one of two processes that use petrochemically derived precursors: (1) a two-step (indirect) process during which propylene is hydrogenated and then hydrolysed using acid and water or (2) a one-step (direct) process during which propylene is hydrogenated using an acid catalyst. IPA is one of the more important solvents used in the chemical industry. It is also an important chemical intermediate. It is a component of cleaners, disinfectants, room sprays, lacquers and thinners, adhesives, pharmaceuticals, cosmetics and toiletries. It is also used as an extractant and as a dehydrating agent. IPA is also used as a gasoline additive, to dissolve water and ice in fuel lines and tanks thereby preventing the water from accumulating in the fuel lines and freezing at low temperatures.

The global demand for isopropanol and propylene continues to increase at a rate of about 3% per year. Since the current methods of producing isopropanol and propylene are primarily manufactured from petroleum, the costs of these compounds will be based on the costs of petroleum. These compounds are also obtained by cracking gasoline or petroleum which is bad for the environment. Accordingly, there is a need for an environmentally friendly and bio-based alternative to the petro-based production process is the production of IPA by fermentation from renewable biomass. However, to be viable and outperform in the current petrochemical IPA market, a fermentative process for the production of IPA must be cost-effective.

WO 2014/140336 discloses a method for producing propanol comprising:
a) exposing gaseous substrates, e.g. CO₂, to a C1-fixing microorganism in a first fermentation zone; and,
b) passing ethanol and/or acetate from step a) to a C3-producing microorganism, in a second fermentation zone, to convert the ethanol and/or acetate to propionate and/or propanol.

### DESCRIPTION OF THE INVENTION

The present invention provides a biotechnological method of producing propanol and/or propionic acid from ethanol in the presence of acetate and carbon dioxide. In particular, the acetate is present at a concentration of at least 1g/L in a medium comprising a propionogen and ethanol. More in particular, the reaction may also comprise the addition of carbon dioxide.

The method of the invention thus comprises a step of (b) contacting at least one propionogen with carbon dioxide and with an aqueous medium comprising ethanol and acetate;
wherein the acetate is maintained at a concentration of at least about 1 g/L in the aqueous medium during the reaction period.

In some embodiments, the present method also includes an additional step of preparing the acetate and ethanol that is used in the main reaction as defined in claim 1.

The presence of acetate in the aqueous medium comprising the propionogen increases the selectivity for formation of propanol and/or propionic acid from ethanol. Therefore, less by-products such as lactate and/or butyrate are formed. There would thus be less waste of the starting materials making the production of propanol and/or propionic acid a most cost effective process. The yield may consequently be higher and/or the more yield may be obtained in a shorter period of time. Further, the desired product, propanol and/or propionic acid, may be easily extracted without complicated purification steps. This further results in cost and time savings.

A microorganism capable of converting ethanol to propanol and/or propionic acid refers to any microorganism that is able to carry out fermentative production of propanol and/or propionic acid. This microorganism is a propionogen. Propionogens are C3-producing microorganisms. In particular, propionogens refer to any microorganism which is capable of converting ethanol and CO₂ from syngas to propionic acid and propanol. The terms "propionogen" or "C3-producing microorganism" refers to microorganisms which, when contacted with a substrate, convert the substrate to propanol and/or propionic acid. These microorganisms may produce the appropriate enzymes intracellularly and/or extracellularly. These propanol and/or propionic acid producing microorganisms may be capable of utilising starting material for propanol and/or propionic acid fermentation that may be waste materials. For instance, CO₂, ethanol, and acetate derived from syngas may be utilized for the propanol and/or propionic acid production. This is particularly advantageous as inexpensive starting materials can be utilized that would originally have been considered waste. This also enables the removal of waste which consequently reduces environmental pollution. In one example, the propionogen according to the present invention may use at least the methylmalonyl-succinate pathway or the lactate-acrylate pathway to produce propionate from acetate and/or alcohol.

In particular, the propionogen used according to the present invention may be selected from the group consisting of *Clostridium neopropionicum, Clostridium propionicum, Pelobacter propionicus, Desulfobulbus propionicus, Syntrophobacter wolinii, Syntrophobacter pfennigii, Syntrophobacter fumaroxidans, Syntrophobacter sulfatireducens, Smithella propionica, Desulfotomaculum thermobenzoicum* subspecies *thermosyntrophicum, Pelotomaculum thermopropionicum,* and *Pelotomaculum schinkii.* More in particular, the propionogen used according to the present invention may be *Clostridium neopropionicum.*

The propionogen may be any eukaryotic or prokaryotic microorganism that may be genetically modified. More in particular, such microorganism may be a strain selected from the group consisting of *Escherichia sp., Erwinia sp., Serratia sp., Providencia sp., Corynebacteria sp., Pseudomonas sp., Leptospira sp., Salmonellar sp., Brevibacteria sp., Hypomononas sp., Chromobacterium sp., Norcardia sp.,* fungi and yeasts. Even more in particular, such microorganism may be selected from *Escherichia sp.* For example, such microorganism may be *Escherichia coli.* Such microorganism may be a genetically modified organism comprising increased expression relative to the wild type cell of propionate CoA-transferase (AJ276553) (E₁), lactoyl-CoA dehydratase (JN244651-3) (E₂) and acryloyl-CoA reductase (JN244654-6) (E₃). Kandasamy V. (2013) discloses a method of producing a genetic organism as such. Kandasamy V. also discloses a means of measuring the expression of enzymes E₁, E₂ and E₃ to determine if any one of these enzymes have increased expression relative to the wild type cell.

According to the present invention, the ethanol and acetate are exogenously produced. This means that the propionogen used according to the present invention may not produce ethanol and/or acetate on its own. In particular, the aqueous medium comprising the propionogen already comprises acetate and ethanol before the propionogen begins to produce propanol and/or propionic acid. More in particular, the ethanol is used as the starting material in the presence of carbon dioxide by the propionogen to produce propanol and/or propionic acid in the presence of acetate. The acetate may not be used up in the production of propanol and/or propionic acid. In particular, the acetate may be used to improve the selectivity of production of propanol and/or propionic acid by the propionogen. The presence of the acetate in the reaction mixture is thus maintained to encourage the production of propanol and/or propionic acid and not the by-products.

Rather than waiting for the cell to produce endogenous acetate, exogenously produced acetate is present initially in step (b) in the aqueous medium. In particular, the term "exogenously produced" acetate and ethanol, as used herein, refers to acetate and ethanol produced or purified in a separate reaction vessel prior to contacting the propionogen to acetate and ethanol produced in step (a), i.e. before step (b). In another example, the term "exogenously produced" acetate and ethanol comprises acetate and ethanol produced by an acetogenic bacterial cell prior to contact with the propionogen in step (b). In one example, the acetate and/or ethanol is removed from the reaction vessel and then brought into contact with the propionogen in a second reaction vessel. In another example, the propionogen and acetogenic cells are in the same reaction vessel. The acetate and/or ethanol may be produced by the acetogenic cell brought into contact with at least one carbon source. In the presence of this exogenous acetate, the propionogen converts the exogenously produced ethanol to propanol and/or propionic acid. More in particular, the concentration of exogenously produced acetate and ethanol may be maintained in the aqueous medium at the value or range of values present initially as long as the reaction catalysed by the propionogen in step b) continues.

The term "acetate" as used herein, refers to both acetic acid and salts thereof (CH₃-COO-), which results inevitably, because as known in the art, since the microorganisms work in an aqueous environment, and there is always a balance between salt and acid present. The ratio of molecular acetic acid to acetate is dependent upon the pH of the system, i.e., at a constant "acetate" concentration, the lower the pH, the higher the molecular acetic acid concentration relative to acetate salt.

The concentration of the acetate in the aqueous medium is a crucial aspect. In particular, the concentration of acetate is at least 1g/L in the aqueous medium. This concentration is maintained in the aqueous medium. In one example, the concentration of acetate is maintained at the concentration mentioned above, for example at least above 1.5g/L, 2g/L, 2.5g/L, 3g/L, 3.5g/L, 4g/L, 4.5g/L, 5g/L, 5.5g/L, 6g/L, 6.5g/L, 7g/L, 7.5g/L, 8g/L, 8.5g/L, 9g/L, 9.5g/L, or 10g/L. In particular, the acetate concentration may be maintained between 1g/L to 10g/L in the aqueous medium. More in particular, the acetate concentration is maintained between 1.5g/L to 7g/L in the aqueous medium. Even more in particular, the acetate concentration may be maintained at about 2g/L in the aqueous medium.

The term 'about' as used herein refers to a variation within 20 percent. In particular, the term "about" as used herein refers to +/- 20%, more in particular, +/-10%, even more in particular, +/-5% of a given measurement or value.

A skilled person would be capable of maintaining the concentration acetate at the desired level by methods known in the art. In particular, the skilled person would regularly measure the concentration of acetate in the aqueous medium and adjust the concentration of acetate accordingly by adding a higher or lower concentration of acetate into the medium. In one example, the acetate may be measured used NMR. In particular, the concentration of acetate may be measured using semi-quantitative ¹H-NMR spectroscopy. As an internal quantification standard sodium trimethylsilylpropionate (T(M)SP) may be used. In another example, the concentration of acetate may be measured using an enzyme kit (Article number: 10148261035) from R-Biopharm following the manufacturer's instructions. In one example, the acetate may be added to the aqueous medium in a continuous flow separately from the continuous feed of the aqueous medium. In another example, acetate may be part of the culture medium that is being topped up. In particular, acetate may be fed to the aqueous medium as part of the nutrient feed or separately. Whichever route is taken to feed acetate to the aqueous medium, a skilled person would understand the means to maintain the concentration of acetate in the aqueous medium. In one example, the acetate concentration in the medium may be maintained by topping up the acetate every about 20 hours of fermentation. In another example, the top up of acetate in the medium may take place every about 5, 10, 15, 20, 25, 30 hours from the beginning of the culturing and/or fermentation process. In another example, the acetate may not necessarily be needed to be topped up as acetate may not be used in the method of producing propanol and/or propionic acid.

The concentration of acetate in the aqueous medium is maintained at at least 1g/L for 100% of the reaction time and may be maintained at higher concentrations for 80% of the reaction period. In another example, the higher concentrations of acetate may be maintained for 50%, 55%, 60%, 65%, 70%, 75%, 85%, 90%, 95% or 100% of the reaction time. In this regard, 'reaction time' refers to the period during with a process takes place. In particular, reaction time refers to the period of time from when a reaction starts to when the reaction ends and/or is completed, i.e. where the substrate is used The substrate is ethanol and carbon dioxide, and the reaction is completed when the ethanol in the fermenter is used up and the reaction stops and no further ethanol and carbon dioxide are fed into the fermenter. Therefore, the reaction time refers to the period from which the fermentation starts (i.e. when the ethanol and carbon dioxide first comes into contact with at least one propionogen in a fermenter in suitable fermentation conditions) to when the fermentation ends (i.e. when there is no more ethanol and carbon dioxide in the fermenter and/or when there is another limiting factor in the fermenter that stops the reaction from continuing). In one example, the reaction period may be for 24hr, 42hr, 72hr, or 96hr. In another example, the reaction period may be for 90, 91, 92, 93, 94, 95, or 97 hours.

The acetate from the mixture following step (b) may be recycled. In particular, this means that the propanol and/or propionic acid formed from step (b) may be allowed to accumulate and then separated by means known in the art. The acetate may thus be maintained in the reaction mixture and recycled. The propanol and/or propionic acid formed from step (b) may be removed in a batchwise mode or in a continuous mode. In the latter case, the propanol and/or propionic acid formed are removed continuously by a separation step known in the art.

The person skilled in the art is familiar with methods that may be used to separate propanol and/or propionic acid from an aqueous medium comprising acetate and/or ethanol. For example extraction may be done using a hydrophobic organic solvent, distillation or the like. A skilled person may easily identify the most suitable means of extracting propanol and/or propionic acid by simple trial and error. In particular, the methods may include that disclosed in Keshav, A., 2009 and Galaction, A.-I, 2012.

The method according to the present invention may comprise an earlier step for producing ethanol and/or acetate, as defined in claim 8. Ethanol and/or acetate may be produced by any means known in the art for exogenously introducing these two compounds to at least one propionogen for the production of propanol and/or propionic acid. In particular, a biotechnological method may be used for the production of ethanol and/or acetate. More in particular, ethanol and/or acetate may be produced by (a) contacting at least one acetogenic bacteria with a carbon source. The carbon source may comprise carbon dioxide and/or carbon monoxide.

For step (a), the source of substrates comprising carbon dioxide and/or carbon monoxide, a skilled person would understand that many possible sources for the provision of CO and/or CO₂ as a carbon source exist. It can be seen that in practice, as the carbon source any gas or any gas mixture can be used which is able to supply the microorganisms with sufficient amounts of carbon, so that acetate and/or ethanol, may be formed from the source of CO and/or CO₂.

Generally for step (a), the cell to be used, which refers to any microorganism according to the present invention, the carbon source comprises at least 50% by weight, at least 70% by weight, particularly at least 90% by weight of CO₂ and/or CO, wherein the percentages by weight - % relate to all carbon sources that are available to the cell according to any aspect of the present invention. The carbon material source may be provided.

Examples of carbon sources in gas forms include exhaust gases such as synthesis gas, flue gas and petroleum refinery gases produced by yeast fermentation or clostridial fermentation. These exhaust gases are formed from the gasification of cellulose-containing materials or coal gasification. In one example, these exhaust gases may not necessarily be produced as by-products of other processes but can specifically be produced for use with a mixed culture.

According to the present invention, the carbon source of step (a) may be synthesis gas. Synthesis gas can for example be produced as a by-product of coal gasification. Accordingly, the microorganism would be capable of converting a substance which is a waste product into a valuable resource.

In another example, synthesis gas may be a by-product of gasification of widely available, low-cost agricultural raw materials for use with the mixed culture according to the present method

There are numerous examples of raw materials that can be converted into synthesis gas, as almost all forms of vegetation can be used for this purpose. In particular, raw materials are selected from the group consisting of perennial grasses such as miscanthus, corn residues, processing waste such as sawdust and the like.

In general, synthesis gas may be obtained in a gasification apparatus of dried biomass, mainly through pyrolysis, partial oxidation and steam reforming, wherein the primary products of the synthesis gas are CO, H₂ and CO₂. Syngas may also be a product of electrolysis. In particular, syngas may be a product of electrolysis of CO₂ and/or water. A skilled person would understand the suitable conditions to carry out electrolysis of CO₂ and/or water to produce syngas comprising CO and/or H₂ in a desired amount.

Usually, a portion of the synthesis gas obtained from the gasification process is first processed in order to optimize product yields, and to avoid formation of tar. Cracking of the undesired tar and CO in the synthesis gas may be carried out using lime and/or dolomite. These processes are described in detail in for example, Reed, 1981.

Mixtures of sources can be used as a carbon source. According to the present invention, a reducing agent, for example hydrogen may be supplied together with the carbon source. In particular, this hydrogen may be supplied when the C and/or CO₂ is supplied and/or used. In one example, the hydrogen gas is part of the synthesis gas present according to the present invention. In another example, where the hydrogen gas in the synthesis gas is insufficient for the method of the present invention, additional hydrogen gas may be supplied.

The term "acetogenic bacteria" as used herein refers to a microorganism which is able to perform the Wood-Ljungdahl pathway and thus is able to convert CO, CO₂ and/or hydrogen to acetate. These microorganisms include microorganisms which in their wild-type form do not have a Wood-Ljungdahl pathway, but have acquired this trait as a result of genetic modification. Such microorganisms include but are not limited to *E. coli* cells. These microorganisms may be also known as carboxydotrophic bacteria. Currently, 21 different genera of the acetogenic bacteria are known in the art (Drake et al., 2006), and these may also include some *clostridia* (Drake & Kusel, 2005). These bacteria are able to use carbon dioxide or carbon monoxide as a carbon source with hydrogen as an energy source (Wood, 1991). Further, alcohols, aldehydes, carboxylic acids as well as numerous hexoses may also be used in step (a) as a carbon source (Drake et al., 2004). The reductive pathway that leads to the formation of acetate is referred to as acetyl-CoA or Wood-Ljungdahl pathway.

In particular, the acetogenic bacteria may be selected from the group consisting of *Acetoanaerobium notera (ATCC 35199), Acetonema longum (DSM 6540), Acetobacterium carbinolicum (DSM 2925), Acetobacterium malicum (DSM 4132), Acetobacterium species no. 446 (*Morinaga et al., 1990, J. Biotechnol., Vol. 14, p. 187-194*),Acetobacterium wieringae (DSM 1911), Acetobacterium woodii (DSM 1030), Alkalibaculum bacchi (DSM 22112), Archaeoglobus fulgidus (DSM 4304), Blautia producta (DSM 2950, formerly Ruminococcus productus, formerly Peptostreptococcus productus), Butyribacterium methylotrophicum (DSM 3468), Clostridium aceticum (DSM 1496), Clostridium autoethanogenum (DSM 10061, DSM 19630 and DSM 23693), Clostridium carboxidivorans (DSM 15243), Clostridium coskatii (ATCC no. PTA-10522), Clostridium drakei (ATCC BA-623), Clostridium formicoaceticum (DSM 92), Clostridium glycolicum (DSM 1288), Clostridium Ijungdahlii (DSM 13528), Clostridium Ijungdahlii C-01 (ATCC 55988), Clostridium Ijungdahlii ERI-2 (ATCC 55380), Clostridium Ijungdahlii O-52 (ATCC 55989), Clostridium mayombei (DSM 6539), Clostridium methoxybenzovorans (DSM 12182), Clostridium ragsdalei (DSM 15248), Clostridium scatologenes (DSM 757), Clostridium species ATCC 29797 (*Schmidt et al., 1986, Chem. Eng. Commun., Vol. 45, p. 61-73*), Desulfotomaculum kuznetsovii (DSM 6115), Desulfotomaculum thermobezoicum subsp. thermosyntrophicum (DSM 14055), Eubacterium limosum (DSM 20543), Methanosarcina acetivorans C2A (DSM 2834), Moorella sp. HUC22-1 (Sakai et al., 2004), Moorella thermoacetica (DSM 521, formerly Clostridium thermoaceticum), Moorella thermoautotrophica (DSM 1974), Oxobacter pfennigii (DSM 322), Sporomusa aerivorans (DSM 13326), Sporomusa ovata (DSM 2662), Sporomusa silvacetica (DSM 10669), Sporomusa sphaeroides (DSM 2875), Sporomusa termitida (DSM 4440) and Thermoanaerobacter kivui (DSM 2030, formerly Acetogenium kivui).* More in particular, the strain ATCC BAA-624 of *Clostridium carboxidivorans* may be used. Even more in particular, the bacterial strain labelled "P7" and "P11" of *Clostridium carboxidivorans* as described for example in U.S. 2007/0275447 and U.S. 2008/0057554 may be used.

Another particularly suitable bacterium may be *Clostridium Ijungdahlii.* In particular, strains selected from the group consisting of *Clostridium Ijungdahlii* PETC, *Clostridium Ijungdahlii* ERI2, *Clostridium Ijungdahlii* COL and *Clostridium Ijungdahlii* 0-52 may be used in the conversion of synthesis gas to hexanoic acid. These strains for example are described in WO 98/00558, WO 00/68407, ATCC 49587, ATCC 55988 and ATCC 55989. The first and second acetogenic bacteria used according to the present invention may be the same or different bacteria. For example, in one reaction mixture the first acetogenic bacteria may be *Clostridium Ijungdahlii* in the log phase and the second acetogenic bacteria may be *Clostridium Ijungdahlii* in the stationary phase. In another example, in the reaction mixture the first acetogenic bacteria may be *Clostridium Ijungdahlii* in the log phase and the second acetogenic bacteria may be *Clostridium carboxidivorans* in the stationary phase.

In the reaction mixture according to the present invention, there may be oxygen present (i.e. aerobic conditions are used). It is advantageous to incorporate O₂ in the reaction mixture and/or gas flow being supplied to the reaction mixture as most waste gases including synthesis gas comprises oxygen in small or large amounts. It is difficult and costly to remove this oxygen prior to using synthesis gas as a carbon source for production of higher alcohols. The method according to the present invention allows the production of at least propanol without the need to first remove any trace of oxygen from the carbon source. This allows for time and money to be saved.

The term "contacting", as used herein, means bringing about direct contact between the cell and the medium comprising the carbon source in step (a) and the direct contact between the propionogen and the acetate and ethanol from step (a) in step (b). For example, the cell, and the medium comprising the carbon source may be in different compartments in step (a). In particular, the carbon source may be in a gaseous state and added to the medium comprising the cells according to the method of the present invention.

In particular, the aqueous medium may comprise the cells and a carbon source comprising CO and/or CO₂ for step (a) to be carried out. More in particular, the carbon source comprising CO and/or CO₂ is provided to the aqueous medium comprising the cells in a continuous gas flow. Even more in particular, the continuous gas flow comprises synthesis gas. These gases may be supplied for example using nozzles that open up into the aqueous medium, frits, membranes within the pipe supplying the gas into the aqueous medium and the like.

The overall efficiency, alcohol productivity and/or overall carbon capture of the method of the present invention may be dependent on the stoichiometry of the CO₂, CO, and H₂ in the continuous gas flow. The continuous gas flows applied may be of composition CO₂ and H₂. In particular, in the continuous gas flow, concentration range of CO₂ may be about 10-50 %, in particular 3 % by weight and H₂ would be within 44 % to 84 %, in particular, 64 to 66.04 % by weight. In another example, the continuous gas flow can also comprise inert gases like N₂, up to a N₂ concentration of 50 % by weight.

According to the present invention, the propionogen and/or the acetogenic bacteria may be a genetically modified microorganism. The genetically modified cell or microorganism may be genetically different from the wild type cell or microorganism. The genetic difference between the genetically modified microorganism according to any aspect of the present invention and the wild type microorganism may be in the presence of a complete gene, amino acid, nucleotide etc. in the genetically modified microorganism that may be absent in the wild type microorganism. In one example, the genetically modified microorganism according to the present invention may comprise enzymes that enable the microorganism to produce propanol and/or propionic acid. The wild type microorganism relative to the genetically modified microorganism according to the present invention may have none or no detectable activity of the enzymes that enable the genetically modified microorganism to produce propanol and/or propionic acid. As used herein, the term 'genetically modified microorganism' may be used interchangeably with the term 'genetically modified cell'. The genetic modification according to the present invention may be carried out on the cell of the microorganism.

The phrase "wild type" as used herein in conjunction with a cell or microorganism may denote a cell with a genome make-up that is in a form as seen naturally in the wild. The term may be applicable for both the whole cell and for individual genes. The term "wild type" therefore does not include such cells or such genes where the gene sequences have been altered at least partially by man using recombinant methods.

A skilled person would be able to use any method known in the art to genetically modify a cell or microorganism. According to the present invention, the genetically modified cell may be genetically modified so that in a defined time interval, within 2 hours, in particular within 8 hours or 24 hours, it forms at least twice, especially at least 10 times, at least 100 times, at least 1000 times or at least 10000 times more propanol and/or propionic acid than the wild-type cell. The increase in product formation can be determined for example by cultivating the cell according to the present invention and the wild-type cell each separately under the same conditions (same cell density, same nutrient medium, same culture conditions) for a specified time interval in a suitable nutrient medium and then determining the amount of target product (propanol and/or propionic acid) in the nutrient medium.

The phrase "increased activity of an enzyme", as used herein is to be understood as increased intracellular activity. Basically, an increase in enzymatic activity can be achieved by increasing the copy number of the gene sequence or gene sequences that code for the enzyme, using a strong promoter or employing a gene or allele that codes for a corresponding enzyme with increased activity and optionally by combining these measures. Genetically modified cells used in the method according to the invention are for example produced by transformation, transduction, conjugation or a combination of these methods with a vector that contains the desired gene, an allele of this gene or parts thereof and a vector that makes expression of the gene possible. Heterologous expression is in particular achieved by integration of the gene or of the alleles in the chromosome of the cell or an extrachromosomally replicating vector. Similarly, a decreased activity of an enzyme refers to decreased intracellular activity. In one example, the increased expression of an enzyme according to the present invention may be 5, 10, 15, 20, 25, 30, 25, 40, 45, 50, 55, 60, 65, 70, 75, 80, 85, 90, 95 or 100% more relative to the expression of the enzyme in the wild type cell. Similarly, the decreased expression of an enzyme according to the present invention may be 5, 10, 15, 20, 25, 30, 25, 40, 45, 50, 55, 60, 65, 70, 75, 80, 85, 90, 95 or 100% less relative to the expression of the enzyme in the wild type cell.

The culture medium to be used must be suitable for the requirements of the particular strains. Descriptions of culture media for various microorganisms are given in "Manual of Methods for General Bacteriology".

All percentages (%) are, unless otherwise specified, mass percent.

A skilled person would understand the other conditions necessary to carry out the method according to the present invention. In particular, the conditions in the container (e.g. fermenter) may be varied depending on the acetogenic bacteria and/or propionogen used. The varying of the conditions to be suitable for the optimal functioning of the microorganisms is within the knowledge of a skilled person.

In one example, the method according to the present invention may be carried out in an aqueous medium with a pH between 5 and 8, 5.5 and 7. The pressure may be between 1 and 10 bar (= 10⁵ - 10⁶ Pa).

A skilled person would understand that it may be necessary to monitor the composition and flow rates of the streams. Control of the composition of the stream can be achieved by varying the proportions of the constituent streams to achieve a target or desirable composition. The composition and flow rate of the stream can be monitored by any means known in the art. In one example, the system is adapted to continuously monitor the flow rates and compositions of the streams and combine them to produce a single blended substrate stream in a continuous gas flow of optimal composition, and means for passing the optimised substrate stream to the cell according to any aspect of the present invention.

Microorganisms which convert CO₂ and/or CO to acetate and/or ethanol in step (a) as defined in claim 6, in particular acetate, as well as appropriate procedures and process conditions for carrying out this metabolic reaction is well known in the art. Such processes are, for example described in WO9800558, WO2000014052 and WO2010115054.

The term "an aqueous solution" or "medium" comprises any solution comprising water, mainly water as solvent that may be used to keep the cell according to the present invention, at least temporarily, in a metabolically active and/or viable state and comprises, if such is necessary, any additional substrates. The person skilled in the art is familiar with the preparation of numerous aqueous solutions, usually referred to as media that may be used to keep inventive cells, for example LB medium in the case of *E. coli,* ATCC1754-Medium may be used in the case of *C*. *Ijungdahlii.* It is advantageous to use as an aqueous solution a minimal medium, *i.e.* a medium of reasonably simple composition that comprises only the minimal set of salts and nutrients indispensable for keeping the cell in a metabolically active and/or viable state, by contrast to complex mediums, to avoid dispensable contamination of the products with unwanted side products. For example, M9 medium may be used as a minimal medium. The cells are incubated with the carbon source sufficiently long enough to produce the desired product, 3HB and variants thereof. For example for at least 1, 2, 4, 5, 10 or 20 hours. The temperature chosen must be such that the cells according to the present invention remains catalytically competent and/or metabolically active, for example 10 to 42 °C, preferably 30 to 40 °C, in particular, 32 to 38 °C in case the cell is a *C*. *Ijungdahlii* cell.

In one example, steps (a) and (b) may be carried it out in a single container. This allows for the accumulation of the propanol and/or propionic acid to take place and less media to be used making the reaction more cost effective. The ethanol produced from step (a) may be used in the production of propanol and/or propionic acid and the acetate produced from step (a) may be used to improve the selectivity of the reaction.

In another example, step (a) and step (b) may be carried out in two different containers. In one example, step (a) may be carried out in fermenter 1 wherein the acetogenic bacteria come in contact with the carbon source to produce acetate and/or ethanol. Ethanol and/or acetate may then be brought into contact with a propionogen in fermenter 2 to produce propanol and/or propionic acid. The propanol and/or propionic acid may then be extracted and the remaining carbon substrate fed back into fermenter 1. A cycle may be created wherein the acetate and/or ethanol produced in fermenter 1 may be regularly fed into fermenter 2, the ethanol in fermenter 2 may be converted to propanol and/or propionic acid and the unreacted carbon source in fermenter 2 fed back into fermenter 1.

In one example, the acetogenic bacteria may be present in a first fermenter and a propionogen in a second fermenter. In fermenter 1, the acetogenic bacteria come in contact with the carbon source to produce acetate and/or ethanol. Ethanol and/or acetate may then be brought into contact with a propionogen in fermenter 2 to produce at least propanol and/or propionic acid. A cycle may be created wherein the acetate and/or ethanol produced in fermenter 1 may be regularly fed into fermenter 2, the acetate and/or ethanol in fermenter 2 may be converted to at least propanol and/or propionic acid. Oxygen may be added into fermenter 2 to enable the propionogen to convert acetate to at least one propanol and/or propionic acid.

Similarly, in fermenter 1 the acetogenic bacteria may come in contact with the carbon source comprising CO to produce acetate and/or ethanol. Ethanol and/or acetate may then be brought into contact with a propionogen in fermenter 2 to produce propanol and/or propionic acid. A cycle may be created wherein the acetate and/or ethanol produced in fermenter 1 may be regularly fed into fermenter 2, the acetate and/or ethanol in fermenter 2 may be converted to propanol and/or propionic acid. CO fed into fermenter 1 may be transferred into fermenter 2 together with the acetate and/or ethanol. No special extraction method may be needed between the two fermenters. In another example, the media is being recycled between fermenters 1 and 2. Therefore, the propanol and/or propionic acid produced in fermenter 2 may be fed back into fermenter 1 to accumulate the propanol, propionic acid and/or derivatives thereof d produced according to the present invention in the fermenters.

### EXAMPLES

### Example 1

### Production of propionic acid and propanol with Clostridium neopropionicum on acetate

For the biotransformation of ethanol and carbon dioxide to propionic acid and propanol the bacterium *Clostridium neopropionicum* was cultivated in mineral medium with ethanol, acetate and a gas atmosphere with carbon dioxide. All cultivation steps were carried out under anaerobic conditions in pressure-resistant glass bottles that can be closed airtight with a butyl rubber stopper. For the first preculture of *C*. *neopropionicum* 2 x 100 ml DSMZ318 medium (pH 7.4; 0.61 g/l NaCl, 0.047 g/l MgCl₂, 0.30 g/l KH₂PO₄, 1.00 g/l NH₄Cl, 0.081 g/l CaCl₂ x 2 H₂O, 0.5 g/l yeast extract, 0.5 g/l BBL Trypticase Peptone, 4 g/L KHCO₃, 1.026 g/L ethanol, 0.5 mg/l resazurin, 128 mg/L nitrilotriacetic acid, 135 mg/L FeCl₃ x 6 H₂O, 1 mg/L MnCl₂ x 4 H₂O, 0.24 mg/L CoCl₂ x 6 H₂O, 1 mg/L ZnCl₂, 0.25 mg/L CuCl₂ x 2 H₂O, 0.1 mg/L H₃BO₃, 0.24 mg/L Na₂MoO₄ x 2 H₂O, 1.2 mg/L NiCl₂ x 6 H₂O, 0.26 mg/L Na₂SeO₃ x 5 H₂O, 0.02 mg/L biotin, 0.02 mg/L folic acid, 0.1 mg/L pyridoxin-HCI, 0.05 mg/L thiamine-HCI x H₂O, 0.05 mg/L riboflavin, 0.05 mg/L nicotinic acid, 0.05 mg/L D-Ca-pantothenate, 1 µg/L vitamin B12, 0.05 mg/L p- aminobenzoate, 0.05 mg/L lipoic acid, 0.25 g/L cysteine-HCI x H₂O) in a 250 ml bottle were inoculated with 5 ml of a frozen cryoculture and flushed with a premixed gas with 67% H₂, 33% CO₂ to an overpressure of 0.8 bar. The culture was incubated at 30°C and 100 rpm in an open water bath shaker for 24 h till an OD₆₀₀ₙₘ >0.15. For a second preculture of C. *neopropionicum* 3 x 200 ml of fresh DSMZ318 medium in a 500 ml bottle were inoculated with centrifuged cells from the first preculture to an OD₆₀₀ₙₘ of 0.03 and flushed with a premixed gas with 67% H₂, 33% CO₂ to an overpressure of 0.8 bar. This growing culture was incubated at 30°C and 100 rpm in an open water bath shaker for 22 h till an OD₆₀₀ₙₘ >0.23.

For the main culture, as many centrifuged cells from the second preculture of *C*. *neopropionicum* as necessary for an OD₆₀₀ₙₘ of 0.2 were added to 200 ml of fresh LM33 mineral medium (pH = 6.8, 10 g/L ethanol, 1 g/L NaOH, 0.5 g/L MgCl₂, 0.21 g/L NaCl, 0.135 g/L CaCl₂ X 2H₂O, 2.65 g/L NaH₂PO₄ X 2H₂O, 0.5 g/L KCI, 2.5 g/L NH₄Cl, 15 mg/L nitrilotriacetic acid, 30 mg/L MgSO₄ x 7 H₂O, 5 mg/L MnSO₄ x H₂O, 1 mg/L FeSO₄ x 7 H₂O, 8 mg/L Fe(SO₄)₂(NH₄)₂ x 6 H₂O, 2 mg/L CoCl₂ x 6 H₂O, 2 mg/L ZnSO₄ x 7 H₂O, 200 µg/L CuCl₂ x 2 H₂O, 200 µg/L KAl(SO₄)₂ x 12 H₂O, 3 mg/L H₃BO₃, 300 µg/L Na₂MoO₄ x 2 H₂O, 200 µg/L Na₂SeO₃, 200 µg/L NiCl₂ x 6 H₂O, 200 µg/L Na₂WO₄ x 6 H₂O, 200 µg/L d-biotin, 200 µg/L folic acid, 100 µg/L pyridoxine-HCI, 500 µg/L thiamine-HCI; 500 µg/L riboflavin; 500 µg/L nicotinic acid; 500 µg/L Ca-pantothenate; 500 µg/L vitamin B₁₂; 500 µg/L p-aminobenzoate; 500 µg/L lipoic acid, 10 mg/L FeCl₃, aerated for 30 min with a premixed gas with 67% H₂ and 33% CO₂) with additional 1.39 g/L sodium acetate. The cultivation was carried out in a 500 mL pressure-resistant glass bottle at 30°C, 100 rpm and an overpressure of 0.8 bar of a premixed gas with 67% H₂, 33% CO₂ in an open water bath shaker for 113 h. The pH was hold at 6.8 by automatic addition of NaOH solution (100 g/L).

During cultivation several 5 mL samples were taken to determinate OD₆₀₀ₙₘ, pH und product formation. The determination of the product concentrations was performed by semi-quantitative ¹H-NMR spectroscopy. As an internal quantification standard sodium trimethylsilylpropionate (T(M)SP) was used.

During the cultivation the concentration of propionate increased from 0.05 g/L to 3.20 g/L, for propanol from 0.01 to 0.26 g/L, for butyrate from 0.01 to 0.33 g/L and for lactate from 0.00 g/L to 0.13 g/L. The concentration of ethanol decreased from 10.1 g/L to 5.2 g/L during this time. The selectivity of propionate/propanol formation was about 88.0%.

### Example 2

### Production of propionic acid and propanol with Clostridium neopropionicum on acetate

For the biotransformation of ethanol and carbon dioxide to propionic acid and propanol the bacterium *Clostridium neopropionicum* was cultivated in mineral medium with ethanol, acetate and a gas atmosphere with carbon dioxide. All cultivation steps were carried out under anaerobic conditions in pressure-resistant glass bottles that can be closed airtight with a butyl rubber stopper. For the first preculture of C. *neopropionicum* 2 x 100 ml DSMZ318 medium (pH 7.4; 0.61 g/l NaCl, 0.047 g/l MgCl₂, 0.30 g/l KH₂PO₄, 1.00 g/l NH₄Cl, 0.081 g/l CaCl₂ x 2 H₂O, 0.5 g/l yeast extract, 0.5 g/l BBL Trypticase Peptone, 4 g/L KHCO₃, 1.026 g/L ethanol, 0.5 mg/l resazurin, 128 mg/L nitrilotriacetic acid, 135 mg/L FeCl₃ x 6 H₂O, 1 mg/L MnCl₂ x 4 H₂O, 0.24 mg/L CoCl₂ x 6 H₂O, 1 mg/L ZnCl₂, 0.25 mg/L CuCl₂ x 2 H₂O, 0.1 mg/L H₃BO₃, 0.24 mg/L Na₂MoO₄ x 2 H₂O, 1.2 mg/L NiCl₂ x 6 H₂O, 0.26 mg/L Na₂SeO₃ x 5 H₂O, 0.02 mg/L biotin, 0.02 mg/L folic acid, 0.1 mg/L pyridoxin-HCI, 0.05 mg/L thiamine-HCI x H₂O, 0.05 mg/L riboflavin, 0.05 mg/L nicotinic acid, 0.05 mg/L D-Ca-pantothenate, 1 µg/L vitamin B12, 0.05 mg/L p- aminobenzoate, 0.05 mg/L lipoic acid, 0.25 g/L cysteine-HCI x H₂O) in a 250 ml bottle were inoculated with 5 ml of a frozen cryoculture and flushed with a premixed gas with 67% H₂, 33% CO₂ to an overpressure of 0.8 bar. The culture was incubated at 30°C and 100 rpm in an open water bath shaker for 24 h till an OD₆₀₀ₙₘ >0.15. For a second preculture of *C*. *neopropionicum* 3 x 200 ml of fresh DSMZ318 medium in a 500 ml bottle were inoculated with centrifuged cells from the first preculture to an OD₆₀₀ₙₘ of 0.03 and flushed with a premixed gas with 67% H₂, 33% CO₂ to an overpressure of 0.8 bar. This growing culture was incubated at 30°C and 100 rpm in an open water bath shaker for 22 h till an OD₆₀₀ₙₘ >0.23.

For the main culture, as many centrifuged cells from the second preculture of C. *neopropionicum* as necessary for an OD₆₀₀ₙₘ of 0.2 were added to 200 ml of fresh LM33 mineral medium (pH = 6.8, 10 g/L ethanol, 1 g/L NaOH, 0.5 g/L MgCl₂, 0.21 g/L NaCl, 0.135 g/L CaCl₂ X 2H₂O, 2.65 g/L NaH₂PO₄ X 2H₂O, 0.5 g/L KCI, 2.5 g/L NH₄Cl, 15 mg/L nitrilotriacetic acid, 30 mg/L MgSO₄ x 7 H₂O, 5 mg/L MnSO₄ x H₂O, 1 mg/L FeSO₄ x 7 H₂O, 8 mg/L Fe(SO₄)₂(NH₄)₂ x 6 H₂O, 2 mg/L CoCl₂ x 6 H₂O, 2 mg/L ZnSO₄ x 7 H₂O, 200 µg/L CuCl₂ x 2 H₂O, 200 µg/L KAl(SO₄)₂ x 12 H₂O, 3 mg/L H₃BO₃, 300 µg/L Na₂MoO₄ x 2 H₂O, 200 µg/L Na₂SeO₃, 200 µg/L NiCl₂ x 6 H₂O, 200 µg/L Na₂WO₄ x 6 H₂O, 200 µg/L d-biotin, 200 µg/L folic acid, 100 µg/L pyridoxine-HCI, 500 µg/L thiamine-HCI; 500 µg/L riboflavin; 500 µg/L nicotinic acid; 500 µg/L Ca-pantothenate; 500 µg/L vitamin B₁₂; 500 µg/L p-aminobenzoate; 500 µg/L lipoic acid, 10 mg/L FeCl₃, aerated for 30 min with a premixed gas with 67% H₂ and 33% CO₂) with additional 6.95 g/L sodium acetate. The cultivation was carried out in a 500 mL pressure-resistant glass bottle at 30°C, 100 rpm and an overpressure of 0.8 bar of a premixed gas with 67% H₂, 33% CO₂ in an open water bath shaker for 113 h. The pH was hold at 6.8 by automatic addition of NaOH solution (100 g/L).

During cultivation several 5 mL samples were taken to determinate OD₆₀₀ₙₘ, pH und product formation. The determination of the product concentrations was performed by semi-quantitative ¹H-NMR spectroscopy. As an internal quantification standard sodium trimethylsilylpropionate (T(M)SP) was used.

During the cultivation the concentration of propionate increased from 0.05 g/L to 3.35 g/L, for propanol from 0.01 to 0.19 g/L, for butyrate from 0.00 to 0.19 g/L and for lactate from 0.00 g/L to 0.16 g/L. The concentration of ethanol decreased from 10.0 g/L to 5.4 g/L during this time. The selectivity of propionate/propanol formation was about 91.0%.

### Example 3

### Production of propionic acid and propanol with Clostridium neopropionicum

For the biotransformation of ethanol and carbon dioxide to propionic acid and propanol the bacterium *Clostridium neopropionicum* was cultivated in mineral medium with ethanol and a gas atmosphere with carbon dioxide. All cultivation steps were carried out under anaerobic conditions in pressure-resistant glass bottles that can be closed airtight with a butyl rubber stopper.

For the first preculture of C. *neopropionicum* 2 x 100 ml DSMZ318 medium (pH 7.4; 0.61 g/l NaCl, 0.047 g/l MgCl₂, 0.30 g/l KH₂PO₄, 1.00 g/l NH₄Cl, 0.081 g/l CaCl₂ x 2 H₂O, 0.5 g/l yeast extract, 0.5 g/l BBL Trypticase Peptone, 4 g/L KHCO₃, 1.026 g/L ethanol, 0.5 mg/l resazurin, 128 mg/L nitrilotriacetic acid, 135 mg/L FeCl₃ x 6 H₂O, 1 mg/L MnCl₂ x 4 H₂O, 0.24 mg/L CoCl₂ x 6 H₂O, 1 mg/L ZnCl₂, 0.25 mg/L CuCl₂ x 2 H₂O, 0.1 mg/L H₃BO₃, 0.24 mg/L Na₂MoO₄ x 2 H₂O, 1.2 mg/L NiCl₂ x 6 H₂O, 0.26 mg/L Na₂SeO₃ x 5 H₂O, 0.02 mg/L biotin, 0.02 mg/L folic acid, 0.1 mg/L pyridoxin-HCI, 0.05 mg/L thiamine-HCI x H₂O, 0.05 mg/L riboflavin, 0.05 mg/L nicotinic acid, 0.05 mg/L D-Ca-pantothenate, 1 µg/L vitamin B12, 0.05 mg/L p- aminobenzoate, 0.05 mg/L lipoic acid, 0.25 g/L cysteine-HCI x H₂O) in a 250 ml bottle were inoculated with 5 ml of a frozen cryoculture and flushed with a premixed gas with 67% H₂, 33% CO₂ to an overpressure of 0.8 bar. The culture was incubated at 30°C and 100 rpm in an open water bath shaker for 24 h till an OD₆₀₀ₙₘ >0.15. For a second preculture of C. *neopropionicum* 3 x 200 ml of fresh DSMZ318 medium in a 500 ml bottle were inoculated with centrifuged cells from the first preculture to an OD₆₀₀ₙₘ of 0.03 and flushed with a premixed gas with 67% H₂, 33% CO₂ to an overpressure of 0.8 bar. This growing culture was incubated at 30°C and 100 rpm in an open water bath shaker for 22 h till an OD₆₀₀ₙₘ >0.23.

For the main culture, as many centrifuged cells from the second preculture of C. *neopropionicum* as necessary for an OD₆₀₀ₙₘ of 0.2 were added to 200 ml of fresh LM33 mineral medium (pH = 6.8, 10 g/L ethanol, 1 g/L NaOH, 0.5 g/L MgCl₂, 0.21 g/L NaCl, 0.135 g/L CaCl₂ X 2H₂O, 2.65 g/L NaH₂PO₄ X 2H₂O, 0.5 g/L KCI, 2.5 g/L NH₄Cl, 15 mg/L nitrilotriacetic acid, 30 mg/L MgSO₄ x 7 H₂O, 5 mg/L MnSO₄ x H₂O, 1 mg/L FeSO₄ x 7 H₂O, 8 mg/L Fe(SO₄)₂(NH₄)₂ x 6 H₂O, 2 mg/L CoCl₂ x 6 H₂O, 2 mg/L ZnSO₄ x 7 H₂O, 200 µg/L CuCl₂ x 2 H₂O, 200 µg/L KAl(SO₄)₂ x 12 H₂O, 3 mg/L H₃BO₃, 300 µg/L Na₂MoO₄ x 2 H₂O, 200 µg/L Na₂SeO₃, 200 µg/L NiCl₂ x 6 H₂O, 200 µg/L Na₂WO₄ x 6 H₂O, 200 µg/L d-biotin, 200 µg/L folic acid, 100 µg/L pyridoxine-HCI, 500 µg/L thiamine-HCI; 500 µg/L riboflavin; 500 µg/L nicotinic acid; 500 µg/L Ca-pantothenate; 500 µg/L vitamin B₁₂; 500 µg/L p-aminobenzoate; 500 µg/L lipoic acid, 10 mg/L FeCl₃, aerated for 30 min with a premixed gas with 67% H₂ and 33% CO₂). The cultivation was carried out in a 500 mL pressure-resistant glass bottle at 30°C, 100 rpm and an overpressure of 0.8 bar of a premixed gas with 67% H₂, 33% CO₂ in an open water bath shaker for 113 h. The pH was hold at 6.8 by automatic addition of NaOH solution (100 g/L).

During cultivation several 5 mL samples were taken to determinate OD₆₀₀ₙₘ, pH und product formation. The determination of the product concentrations was performed by semi-quantitative ¹H-NMR spectroscopy. As an internal quantification standard sodium trimethylsilylpropionate (T(M)SP) was used.

During the cultivation the concentration of propionate increased from 0.05 g/L to 2.45 g/L, for propanol from 0.01 to 0.45 g/L, for butyrate from 0.01 to 0.32 g/L and for lactate from 0.00 g/L to 0.13 g/L. The concentration of ethanol decreased from 10.5 g/L to 5.4 g/L during this time. The selectivity of propionate/propanol formation was about 86.5%.

### Example 4 (comparative example)

### Production of propionate with Desulfobulbus propionicus

For the biotransformation of ethanol and carbon dioxide to propionic acid the bacterium *Desulfobulbus propionicus* was cultivated in medium with ethanol and a gas atmosphere with carbon dioxide. All cultivation steps were carried out under anaerobic conditions in pressure-resistant glass bottles that can be closed airtight with a butyl rubber stopper.

For the first preculture of *Desulfobulbus propionicus,* 50 ml DSMZ194-medium (3 g/L Na₂SO₄; 0.2 g/L KH₂PO₄; 0.3 g/L NH₄Cl; 1 g/L NaCl; 0.4 g/L MgCl₂ x 6 H₂O; 0.5 g/L KCI; 0.15 g/L CaCl₂ x 2 H₂O; 0.5 mg/L NaOH; 0.003 mg/L Na₂SeO₃ x 5 H₂O; 0.004 mg/L Na₂WO₄ x 2 H₂O; 1 mg/L resazurin; 5 g/L NaHCO₃; 1.5 g/L Na-propionate; 0.4 g/L Na₂S x 9 H₂O; 2.8 mg/L HCl; 1.5 mg/L FeCl₂ x 4 H₂O; 0.07 mg/L ZnCl₂ x 7 H₂O; 0.1 mg/L MnCl₂ x 4 H₂O; 0.006 mg/L H₃BO₃ ; 0.19 mg/L CoCl₂ x 6 H₂O; 0.002 mg/L CuCl₂ x 6 H₂O; 0.024 mg/L NiCl₂ x 6 H₂O; 0.036 mg/L Na₂MO₄ x 2 H₂O; 0.02 mg/L biotin; 0.02 mg/L folic acid; 0.1 mg/L pyridoxine-HCI; 0.05 mg/L thiamine-HCI; 0.05 mg/L riboflavin; 0.05 mg/L nicotinic acid; 0.05 mg/L D-Ca-pantothenate; 0,001 mg/L vitamin B₁₂; 0.05 mg/L p-aminobenzoic acid; 0.05 mg/L lipoic acid) with additional 400 mg/L Na₂S and 420 mg/L KOH were inoculated with 5 mL of a frozen cryo stock. The cultivation was carried out in a 250 mL pressure-resistant glass bottle at 37°C, and an overpressure of 0.8 bar of a premixed gas with 67% N₂ and 33% CO₂ in a heat cabinet without shaking for 72 h till OD₆₀₀ₙₘ of 0.18.

For the second preculture 2 x 100 ml DSMZ194-medium with additional 400 mg/L Na₂S and 420 mg/L KOH were inoculated with centrifuged cells from the first preculture to an OD₆₀₀ₙₘ of 0.038 and 0.034, respectively. The cultivation was carried out in 2 x 250 mL pressure-resistant glass bottles at 37°C, and an overpressure of 0.8 bar of a premixed gas with 67% N₂ and 33% CO₂ for 47 h, one in a heat cabinet without shaking, the other in an open water bath shaker at 100 rpm till OD₆₀₀ₙₘ of 0.2 and 0.18, respectively.

For the main culture 100 ml ATCC1754-medium (pH = 6.0; 20 g/L MES; 1 g/L yeast extract, 0.8 g/L NaCl; 1 g/L NH₄Cl; 0.1 g/L KCI; 0.1 g/L KH₂PO₄; 0.2 g/L MgSO₄ x 7 H₂O; 0.02 g/L CaCl₂ x 2 H₂O; 20 mg/L nitrilotriacetic acid; 10 mg/L MnSO₄ x H₂O; 8 mg/L (NH₄)₂Fe(SO₄)₂ x 6 H₂O; 2 mg/L CoCl₂ x 6 H₂O; 2 mg/L ZnSO₄ x 7 H₂O; 0.2 mg/L CuCl₂ x 2 H₂O; 0.2 mg/L Na₂MoO₄ x 2 H₂O; 0.2 mg/L NiCl₂ x 6 H₂O; 0.2 mg/L Na₂SeO₄; 0.2 mg/L Na₂WO₄ x 2 H₂O; 20 µg/L d-biotin; 20 µg/L folic acid; 100 µg/L pyridoxine-HCI; 50 µg/L thiamine-HCI x H₂O; 50 µg/L riboflavin; 50 µg/L nicotinic acid; 50 µg/L Ca-pantothenate; 1 µg/L vitamin B₁₂; 50 µg/L p-aminobenzoate; 50 µg/L lipoic acid; approx. 67.5 mg/L NaOH) with additional 400 mg/L L-cysteine-hydrochloride, 5 mL/L ethanol and 5.74 g/L KOH, were inoculated with centrifuged cells from both second precultures to an OD₆₀₀ₙₘ of 0.09. The cultivation was started at a pH of 7.0.

The cultivation was carried out in a 500 mL pressure-resistant glass bottle at 37°C, 100 rpm and an overpressure of 0.8 bar of a premixed gas with 67% N₂ and 33% CO₂ in an open water bath shaker for 170 h till OD₆₀₀ₙₘ of 0.07 and pH 6.8.

During cultivation several 5 mL samples were taken to determinate OD₆₀₀ₙₘ, pH and product formation. The determination of the product concentrations was performed by semi-quantitative ¹H-NMR spectroscopy. As an internal quantification standard sodium trimethylsilylpropionate (T(M)SP) was used.

During the main cultivation the concentration of acetate increased from 0.01 g/L to 0.45 g/L, for propionate from 0.00 g/L to 0.3 g/L, and for propanol from 0.00 g/L to 0.16 g/L. The concentration of ethanol decreased from 4.85 g/L to 3.95 g/L.

### Example 5

### Production of propionate with Clostridium propionicum

For the biotransformation of ethanol and carbon dioxide to propionic acid the bacterium *Clostridium propionicum* was cultivated in medium with ethanol and a gas atmosphere with carbon dioxide. All cultivation steps were carried out under anaerobic conditions in pressure-resistant glass bottles that can be closed airtight with a butyl rubber stopper.

For the first preculture 50 ml DSMZ156-medium (3.0 g/L L-Alanine; 3.0 g/L peptone; 4.0 g/L yeast extract; 0.1 g/L MgSO₄ x 7 H₂O; 18.0 mg/l FeSO₄ x 7 H₂O; 5.0 mmol/L phosphate buffered saline pH 7.1; 2.5 mL/L saturated calcium sulfate solution; 1.0 mg/L resazurin; 1 g/L NaHCO₃) with additional 400 mg/L L-cysteine-hydrochlorid were inoculated with 5 mL of a frozen cryo stock. The cultivation was carried out in a 250 mL pressure-resistant glass bottle at 37°C, 100 rpm and an overpressure of 0.8 bar of a premixed gas with 67% N₂ and 33% CO₂ in an open water bath shaker for 21 h till OD₆₀₀ₙₘ of 0.64.

For the second preculture 100 ml DSMZ156-medium with additional 400 mg/L L-cysteine-hydrochlorid were inoculated with centrifuged cells from the first preculture to an OD₆₀₀ₙₘ of 0.07. The cultivation was carried out in a 250 mL pressure-resistant glass bottle at 37°C, 100 rpm and an overpressure of 0.8 bar of a premixed gas with 67% N₂ and 33% CO₂ in an open water bath shaker for 47 h till OD₆₀₀ₙₘ of 0.69.

For the main culture 100 ml ATCC1754-medium (pH = 6.0; 20 g/L MES; 1 g/L yeast extract, 0.8 g/L NaCl; 1 g/L NH₄Cl; 0.1 g/L KCI; 0.1 g/L KH₂PO₄; 0.2 g/L MgSO₄ x 7 H₂O; 0.02 g/L CaCl₂ x 2 H₂O; 20 mg/L nitrilotriacetic acid; 10 mg/L MnSO₄ x H₂O; 8 mg/L (NH₄)₂Fe(SO₄)₂ x 6 H₂O; 2 mg/L CoCl₂ x 6 H₂O; 2 mg/L ZnSO₄ x 7 H₂O; 0.2 mg/L CuCl₂ x 2 H₂O; 0.2 mg/L Na₂MoO₄ x 2 H₂O; 0.2 mg/L NiCl₂ x 6 H₂O; 0.2 mg/L Na₂SeO₄; 0.2 mg/L Na₂WO₄ x 2 H₂O; 20 µg/L d-biotin; 20 µg/L folic acid; 100 µg/L pyridoxine-HCI; 50 µg/L thiamine-HCI x H₂O; 50 µg/L riboflavin; 50 µg/L nicotinic acid; 50 µg/L Ca-pantothenate; 1 µg/L vitamin B₁₂; 50 µg/L p-aminobenzoate; 50 µg/L lipoic acid; approx. 67.5 mg/L NaOH) with additional 400 mg/L L-cysteine-hydrochlorid, 5 mL/L ethanol and 2.35 g/L KOH, were inoculated with centrifuged cells from the second preculture to an OD₆₀₀ₙₘ of 0.166. The cultivation was started at a pH of 6.6.

The cultivation was carried out in a 500 mL pressure-resistant glass bottle at 37°C, 100 rpm and an overpressure of 0.8 bar of a premixed gas with 67% N₂ and 33% CO₂ in an open water bath shaker for 165 h till OD₆₀₀ₙₘ of 0.21 and pH 6.0.

During cultivation several 5 mL samples were taken to determinate OD₆₀₀ₙₘ, pH and product formation. The determination of the product concentrations was performed by semiquantitative ¹H-NMR spectroscopy. As an internal quantification standard sodium trimethylsilylpropionate (T(M)SP) was used.

During the main cultivation the concentration of acetate increased from 0.01 g/L to 1.15 g/L, for propionate from 0.02 g/L to 1.05 g/L, for propanol from 0.00 to 0.39 g/L, and for butyrate from 0 to 110 mg/L. The concentration of ethanol decreased from 4.40 g/L to 2.75 g/L and for alanine from 34 mg/L to 0 mg/L during this time.

### Example 6

### Production of propionate with Pelobacter propionicus

For the biotransformation of ethanol and carbon dioxide to propionic acid the bacterium *Pelobacter propionicus* was cultivated in medium with ethanol and a gas atmosphere with carbon dioxide. All cultivation steps were carried out under anaerobic conditions in pressure-resistant glass bottles that can be closed airtight with a butyl rubber stopper.

For the first preculture 50 ml DSMZ295-medium (0.2 g/L KH₂PO₄; 0.25 g/L NH₄Cl; 1 g/L NaCl; 0.4 g/L MgCl₂ x 6 H₂O; 0.5 g/L KCI; 0.15 g/L CaCl₂ x 2 H₂O; 2.8 mg/L HCl; 1.5 mg/L FeCl₂ x 4 H₂O; 0.07 mg/L ZnCl₂ x 7 H₂O; 0.1 mg/L MnCl₂ x 4 H₂O; 0.006 mg/L H₃BO₃; 0.19 mg/L CoC₂ x 6 H₂O; 0.002 mg/L CuCl₂ x 6 H₂O; 0.024 mg/L NiCl₂ x 6 H₂O; 0.036 mg/L Na₂MO₄ x 2 H₂O; 1 mg/L resazurin; 2.5 g/L NaHCO₃; 0.9 g/L 2,3-butanediol) with additional 360 mg/L Na₂S were inoculated with 5 mL of a frozen cryo stock. The cultivation was carried out in a 250 mL pressure-resistant glass bottle, at 30°C, 100 rpm in an open water bath shaker and an overpressure of 0.8 bar of a premixed gas with 67% N₂ and 33% CO₂ for 69 h till OD₆₀₀ₙₘ of 0.19 and pH 6.2.

For the second preculture 2 x 100 ml DSMZ295-medium with additional 360 mg/L Na₂S and 420 mg/L KOH were inoculated with centrifuged cells from the first preculture to an OD₆₀₀ₙₘ of 0.055 and 0.053, respectively. The cultivation was carried out in 2 x 250 mL pressure-resistant glass bottles at 30°C, 100 rpm in an open water bath shaker and an overpressure of 0.8 bar of a premixed gas with 67% N₂ and 33% CO₂ for 72 h till OD₆₀₀ₙₘ of 0.24 and pH 6.6 and OD₆₀₀ₙₘ of 0.23 and pH 6.4, respectively.

For the main culture 100 ml modified DSMZ318-medium (pH 7.4; 0.61 g/L NaCl, 0.047 g/L MgCl₂, 0.30 g/L KH₂PO₄, 1.00 g/L NH₄Cl, 0.081 g/L CaCl₂ x 2 H₂O, 0.5 g/L yeast extract, 0.5 g/L BBL Trypticase Peptone, 4 g/L KHCO₃, 5 g/L ethanol, 0.5 mg/L resazurin, 128 mg/L nitrilotriacetic acid, 135 mg/L FeCl₃ x 6 H₂O, 1 mg/L MnCl₂ x 4 H₂O, 0.24 mg/L CoCl₂ x 6 H₂O, 1 mg/L ZnCl₂, 0.25 mg/L CuCl₂ x 2 H₂O, 0.1 mg/L H₃BO₃, 0.24 mg/L Na₂MoO₄ x 2 H₂O, 1.2 mg/L NiCl₂ x 6 H₂O, 0.26 mg/L Na₂SeO₃ x 5 H₂O, 0.02 mg/L biotin, 0.02 mg/L folic acid, 0.1 mg/L pyridoxin-HCI, 0.05 mg/L thiamine-HCI x H₂O, 0.05 mg/L riboflavin, 0.05 mg/L nicotinic acid, 0.05 mg/L D-Ca-pantothenate, 1 µg/L vitamin B12, 0.05 mg/L p- aminobenzoate, 0.05 mg/L lipoic acid) with additional 0.4 g/L L-cysteine-hydrochlorid, 0.7 g/L KOH, and 11.2 mg/L HCl were inoculated with centrifuged cells from both second precultures to an OD₆₀₀ₙₘ of 0.10. The cultivation was started at a pH of 6.8. The cultivation was carried out in a 500 mL pressure-resistant glass bottle at 30°C, 100 rpm and an overpressure of 0.8 bar of a premixed gas with 67% N₂ and 33% CO₂ in an open water bath shaker for 162 h till OD₆₀₀ₙₘ of 0.13 and pH 6.5.

During cultivation several 5 mL samples were taken to determinate OD₆₀₀ₙₘ, pH and product formation. The determination of the product concentrations was performed by semiquantitative ¹H-NMR spectroscopy. As an internal quantification standard sodium trimethylsilylpropionate (T(M)SP) was used.

During the main cultivation the concentration of acetate increased from 0.02 g/L to 0.45 g/L, for propionate from 0.02 to 0.27 g/L, and for propanol from 0.00 to 0.18 g/L. The concentration of ethanol decreased from 4.6 g/L to 4.00 g/L.

### Example 7

### Production of propionic acid and propanol with Clostridium neopropionicum on acetate

For the biotransformation of ethanol and carbon dioxide to propionic acid and propanol the bacterium *Clostridium neopropionicum* was cultivated in mineral medium with ethanol, acetate and a gas atmosphere with carbon dioxide. All cultivation steps were carried out under anaerobic conditions in pressure-resistant glass bottles that can be closed airtight with a butyl rubber stopper. For the first preculture of C. *neopropionicum* 500 mL DSMZ318 medium (pH 7.4; 0.61 g/L NaCl; 0.047 g/L MgCl₂; 0.30 g/L KH₂PO₄; 1.00 g/L NH₄Cl; 0.081 g/L CaCl₂ x 2 H₂O; 0.5 g/L yeast extract; 0.5 g/L BBL Trypticase Peptone; 4 g/L KHCO₃; 10 g/L ethanol; 0.5 mg/L resazurin; 128 mg/L nitrilotriacetic acid; 135 mg/L FeCl₃ x 6 H₂O; 1 mg/L MnCl₂ x 4 H₂O; 0.24 mg/L CoCl₂ x 6 H₂O; 1 mg/L ZnCl₂; 0.25 mg/L CuCl₂ x 2 H₂O; 0.1 mg/L H₃BO₃; 0.24 mg/L Na₂MoO₄ x 2 H₂O; 1.2 mg/L NiCl₂ x 6 H₂O; 0.26 mg/L Na₂SeO₃ x 5 H₂O; 0.02 mg/L biotin; 0.02 mg/L folic acid; 0.1 mg/L pyridoxin-HCI; 0.05 mg/L thiamine-HCI x H₂O; 0.05 mg/L riboflavin; 0.05 mg/L nicotinic acid; 0.05 mg/L D-Ca-pantothenate; 1 µg/L vitamin B₁₂; 0.05 mg/L p- aminobenzoate; 0.05 mg/L lipoic acid; 0.25 g/L cysteine-HCI x H₂O) in a 1 L bottle were inoculated with 5 ml of a frozen cryoculture and flushed with a premixed gas with 67% H₂, 33% CO₂ to an overpressure of 0.8 bar. The culture was incubated at 30°C and 100 rpm in an open water bath shaker for 67 h till an OD₆₀₀ₙₘ 0.17.

For the second preculture of C. *neopropionicum* 500 mL of fresh DSMZ318 medium in a 1 L bottle were inoculated with centrifuged cells from the first preculture to an OD₆₀₀ₙₘ of 0.05 and flushed with a premixed gas with 67% H₂, 33% CO₂ to an overpressure of 0.8 bar. This growing culture was incubated at 30°C and 100 rpm in an open water bath shaker for 24 h till OD₆₀₀ₙₘ 0.13.

For the third preculture of C. *neopropionicum* 2 x 500 mL of fresh DSMZ318 medium in 1 L bottles were inoculated with centrifuged cells from the second preculture to an OD₆₀₀ₙₘ of 0.05 and flushed with a premixed gas with 67% H₂, 33% CO₂ to an overpressure of 0.8 bar. These growing cultures were incubated at 30°C and 100 rpm in an open water bath shaker for 18 h till OD₆₀₀ₙₘ 0.26 and 0.25, respectively.

For the fourth preculture of C. *neopropionicum* 10 x 500 mL of fresh DSMZ318 medium in 1 L bottles were inoculated with centrifuged cells from the third preculture to an OD₆₀₀ₙₘ of 0.03 - 0.04 and flushed with a premixed gas with 67% H₂, 33% CO₂ to an overpressure of 0.8 bar. These growing cultures were incubated at 30°C and 100 rpm in an open water bath shaker for 22 h till OD₆₀₀ₙₘ >0.23

For the main culture, as many centrifuged cells from the fourth preculture of C. *neopropionicum* as necessary for an OD₆₀₀ₙₘ of 0.75 were added to 50 ml of fresh LM33 mineral medium (pH = 6.8, 10 g/L ethanol; 1 g/L NaOH; 0.5 g/L MgCl₂; 0.21 g/L NaCl; 0.135 g/L CaCl₂ X 2H₂O; 2.65 g/L NaH₂PO₄X2 H₂O; 0.5 g/L KCI; 2.5 g/L NH₄Cl; 15 mg/L nitrilotriacetic acid; 30 mg/L MgSO₄ x 7 H₂O; 5 mg/L MnSO₄ x H₂O; 1 mg/L FeSO₄ x 7 H₂O; 8 mg/L Fe(SO₄)₂(NH₄)₂ x 6 H₂O; 2 mg/L CoCl₂ x 6 H₂O; 2 mg/L ZnSO₄ x 7 H₂O; 200 µg/L CuCl₂ x 2 H₂O; 200 µg/L KAI(SO₄)₂ x 12 H₂O; 3 mg/L H₃BO₃; 300 µg/L Na₂MoO₄ x 2 H₂O; 200 µg/L Na₂SeO₃; 200 µg/L NiCl₂ x 6 H₂O; 200 µg/L Na₂WO₄ x 6 H₂O; 200 µg/L d-biotin; 200 µg/L folic acid; 100 µg/L pyridoxine-HCI; 500 µg/L thiamine-HCI; 500 µg/L riboflavin; 500 µg/L nicotinic acid; 500 µg/L Ca-pantothenate; 500 µg/L vitamin B₁₂; 500 µg/L p-aminobenzoate; 500 µg/L lipoic acid; 10 mg/L FeCl₃, aerated for 30 min with CO₂) with additional 13.9 g/L sodium acetate. The cultivation was carried out in a 250 mL pressure-resistant glass bottle at 30°C, 150 rpm and an overpressure of 0.3 bar of CO₂ in an open water bath shaker for 5 h. The pH decreased during this time from 7.0 to 6.8. The OD₆₀₀ₙₘ decreased from 0.75 to 0.71.

During cultivation several 5 mL samples were taken to determinate OD₆₀₀ₙₘ, pH and product formation. The determination of the product concentrations was performed by semiquantitative ¹H-NMR spectroscopy. As an internal quantification standard sodium trimethylsilylpropionate (T(M)SP) was used.

During the cultivation the concentration of propionate increased from 0.03 g/L to 0.77 g/L, for propanol from 0.01 to 0.02 g/L and for lactate from 0.00 g/L to 0.08 g/L. No butyrate was formed. The concentration of ethanol decreased from 9.25 g/L to 8.04 g/L during this time. The selectivity of propionate/propanol formation was about 92.1%.

### REFERENCES

Drake et al., 2004. Strict and Facultative Anaerobes: Medical and Environmental Aspects. pp. 251-281, Horizon Scientific Press, United Kingdom
Drake & Kusel, 2005 Acetogenic clostridia. In: Dürre, P. (ed.), Handbook on Clostridia, pp. 719-746. CRC Press, Boca Raton, Florida.
Drake et al., 2006, Acetogenic prokaryotes. In: Balows A, Trüper HG, Dworkin M, Harder W and Gerhardt, P et al (ed) American Society for Microbiology, Washington, DC. p. 248-277
Fuchs G., Schlegel H.-G. (2007) Allgemeine Mikrobiologie, Georg Thieme Verlag, Stuttgart. Galaction, A.-I. et al., (2012) Chemical Engineering & Technology, 35(9), 1657-1663
Kandasamy V., 2013, Appl Microbial Biotechnol: 97:1191-1200)
Keshav, Amit et al., Desalination (2009), 244(1-3), 12-23
Sakai et al., 2004 Biotechnol. Let., Vol. 29, p. 1607-1612
Wood, 1991 Life with CO or CO2 and H2 as a source of carbon and energy. FASEB J. 5:156-163 Zhang Y, 2013, Bioprocess Biosyst Eng; 36(12):1897-1904
U.S. 2007/0275447, U.S. 2008/0057554, WO 98/00558, WO 00/68407

## Claims

1. A method of producing propanol and/or propionic acid, the method comprising:
(b) contacting at least one propionogen with carbon dioxide and with an aqueous medium comprising ethanol and acetate;
wherein the acetate is maintained at a concentration of at least 1 g/L in the aqueous medium during the reaction period.

2. The method according to claim 1, wherein the ethanol and acetate are exogenously produced.

3. The method according to either claim 1 or 2, wherein the propionogen is selected from the group consisting of *Clostridium neopropionicum*, *Clostridium propionicum*, *Pelobacter propionicus, Desulfobulbus propionicus, Syntrophobacter wolinii, Syntrophobacter pfennigii*, *Syntrophobacter fumaroxidans*, *Syntrophobacter sulfatireducens*, *Smithella propionica*, *Desulfotomaculum thermobenzoicum* subspecies *thermosyntrophicum, Pelotomaculum thermopropionicum,* and *Pelotomaculum schinkii.*

4. The method according to any one of the preceding claims, wherein the propionogen is *Clostridium neopropionicum.*

5. The method according to any one of the preceding claims, wherein the acetate concentration is maintained between 1g/L and 10g/L in the aqueous medium.

6. The method according to any one of the preceding claims, wherein the acetate concentration is maintained between 1.5g/L and 7g/L in the aqueous medium.

7. The method according to any one of the preceding claims, wherein the acetate concentration is maintained at about 2g/L in the aqueous medium, wherein 'about' refers to a variation within 20 percent.

8. The method according to any one of the preceding claims, wherein the method comprises a step (a) for producing ethanol and acetate, wherein step (a) comprises:
(a) contacting at least one acetogenic bacterium with a carbon source, wherein the carbon source comprises carbon dioxide and/or carbon monoxide.

9. The method according to claim 8, wherein the acetogenic bacterium is selected from the group consisting of *Clostridium autothenogenum DSMZ 19630*, *Clostridium ragsdahlei ATCC no. BAA-622, Clostridium autoethanogenum*, *Moorella sp HUC22-1, Moorella thermoaceticum, Moorella thermoautotrophica*, *Rumicoccus productus*, *Acetoanaerobum*, *Oxobacter pfennigii*, *Methanosarcina barkeri*, *Methanosarcina acetivorans, Carboxydothermus, Desulfotomaculum kutznetsovii, Pyrococcus, Peptostreptococcus, Butyribacterium methylotrophicum ATCC 33266*, *Clostridium formicoaceticum*, *Clostridium butyricum*, *Lactobacillus delbrukii*, *Propionibacterium acidoproprionici*, *Proprionispera arboris*, *Anaerobierspirillum succiniproducens, Bacterioides amylophilus, Becterioides ruminicola, Thermoanaerobacter kivui, Acetobacterium woodii, Acetoanaerobium notera, Clostridium aceticum, Butyribacterium methylotrophicum*, *Moorella thermoacetica, Eubacterium limosum, Peptostreptococcus productus, Clostridium Ijungdahlii*, *Clostridium ATCC* 29797 and *Clostridium carboxidivorans.*

10. The method according to claim 8 or 9, wherein steps (a) and (b) are carried out in a single fermenter.

11. A use of acetate for increasing the proportion of ethanol converted to propanol and/or propionic acid by a propionogen in an aqueous medium, wherein acetate in the aqueous medium is maintained at a concentration of at least 1 g/L during the reaction period, wherein the propionogen is contacted with carbon dioxide and with the aqueous medium comprising the ethanol and the acetate.

12. The use according to claim 11, wherein the ethanol and acetate are exogenously produced ethanol and acetate.

13. The use according to either claim 11 or 12, wherein the propionogen is selected from the group consisting of *Clostridium neopropionicum*, *Clostridium propionicum*, *Pelobacter propionicus, Desulfobulbus propionicus, Syntrophobacter wolinii, Syntrophobacter pfennigii*, *Syntrophobacter fumaroxidans, Syntrophobacter sulfatireducens, Smithella propionica*, *Desulfotomaculum thermobenzoicum* subspecies *thermosyntrophicum, Pelotomaculum thermopropionicum,* and *Pelotomaculum schinkii.*

14. The use of any one of claims 11 to 13, wherein the acetate concentration is maintained between 1.5g/L and 7g/L in the aqueous medium.

15. The use according to any one of claims 11 to 14, wherein the acetate concentration is maintained at about 2g/L in the aqueous medium, wherein 'about' refers to a variation within 20 percent.

## Patentansprüche

1. Verfahren zur Herstellung von Propanol und/oder Propionsäure, wobei das Verfahren umfasst:
(b) Inkontaktbringen wenigstens eines Propionogens mit Kohlendioxid und mit einem wässrigen Medium, das Ethanol und Acetat umfasst;
wobei das Acetat während des Reaktionszeitraums bei einer Konzentration von wenigstens 1 g/l in dem wässrigen Medium gehalten wird.

2. Verfahren gemäß Anspruch 1, wobei das Ethanol und das Acetat exogen hergestellt sind.

3. Verfahren gemäß Anspruch 1 oder 2, wobei das Propionogen ausgewählt ist aus der Gruppe bestehend aus *Clostridium neopropionicum, Clostridium propionicum, Pelobacter propionicus, Desulfobulbus propionicus, Syntrophobacter wolinii, Syntrophobacter pfennigii, Syntrophobacter fumaroxidans, Syntrophobacter sulfatireductens, Smithella propionica, Desulfotomaculum thermobenzoicum* subspezies *thermosyntrophicum, Pelotomaculum thermopropionicum* und *Pelotomaculum schinkii.*

4. Verfahren gemäß einem der vorstehenden Ansprüche, wobei das Propionogen *Clostridium neopropionicum* ist.

5. Verfahren gemäß einem der vorstehenden Ansprüche, wobei die Acetatkonzentration bei zwischen 1 g/l und 10 g/l in dem wässrigen Medium gehalten wird.

6. Verfahren gemäß einem der vorstehenden Ansprüche, wobei die Acetatkonzentration bei zwischen 1,5 g/l und 7 g/l in dem wässrigen Medium gehalten wird.

7. Verfahren gemäß einem der vorstehenden Ansprüche, wobei die Acetatkonzentration bei etwa 2 g/l in dem wässrigen Medium gehalten wird, wobei "etwa" eine Variation innerhalb von 20 Prozent bezeichnet.

8. Verfahren gemäß einem der vorstehenden Ansprüche, wobei das Verfahren einen Schritt (a) zur Herstellung von Ethanol und Acetat umfasst, wobei Schritt (a) umfasst:
(a) Inkontaktbringen wenigstens eines acetogenen Bakteriums mit einer Kohlenstoffquelle, wobei die Kohlenstoffquelle Kohlendioxid und/oder Kohlenmonoxid umfasst.

9. Verfahren gemäß Anspruch 8, wobei das acetogene Bakterium ausgewählt ist aus der Gruppe bestehend aus *Clostridium autothenogenum DSMZ 19630, Clostridium ragsdahlei ATCC Nr. BAA-622, Clostridium autoethanogenum, Moorella sp HUC22-1, Moorella thermoaceticum, Moorella thermoautotrophica, Rumicoccus productus, Acetoanaerobum, Oxobacter pfennigii, Methanosarcina barkeri, Methanosarcina acetivorans, Carboxydothermus, Desulfotomaculum kutznetsovii, Pyrococcus, Peptostreptococcus, Butyribacterium methylotrophicum ATCC 33266, Clostridium formicoaceticum, Clostridium butyricum, Lactobacillus delbrukii, Propionibacterium acidoproprionici, Proprionispera arboris, Anaerobierspirillum succiniproducens, Bacterioides amylophilus, Becterioides ruminicola, Thermoanaerobacter kivui, Acetobacterium woodii, Acetoanaerobium notera, Clostridium aceticum, Butyribacterium methylotrophicum, Moorella thermoacetica, Eubacterium limosum, Peptostreptococcus productus, Clostridium Ijungdahlii, Clostridium ATCC 29797* und *Clostridium carboxidivorans.*

10. Verfahren gemäß Anspruch 8 oder 9, wobei die Schritte (a) und (b) in einem einzigen Fermenter durchgeführt werden.

11. Verwendung von Acetat zum Erhöhen des Anteils von Ethanol, das von einem Propionogen in einem wässrigen Medium zu Propanol und/oder Propionsäure umgewandelt wird, wobei das Acetat während des Reaktionszeitraums bei einer Konzentration von wenigstens 1 g/l in dem wässrigen Medium gehalten wird, wobei das Propionogen mit Kohlendioxid und mit dem wässrigen Medium, das das Ethanol und das Acetat umfasst, in Kontakt gebracht wird.

12. Verwendung gemäß Anspruch 11, wobei das Ethanol und das Acetat exogen hergestelltes Ethanol und Acetat sind.

13. Verwendung gemäß Anspruch 11 oder 12, wobei das Propionogen ausgewählt ist aus der Gruppe bestehend aus *Clostridium neopropionicum, Clostridium propionicum, Pelobacter propionicus, Desulfobulbus propionicus, Syntrophobacter wolinii, Syntrophobacter pfennigii, Syntrophobacter fumaroxidans, Syntrophobacter sulfatireductens, Smithella propionica, Desulfotomaculum thermobenzoicum* subspezies *thermosyntrophicum, Pelotomaculum thermopropionicum* und *Pelotomaculum schinkii.*

14. Verwendung gemäß einem der Ansprüche 11 bis 13, wobei die Acetatkonzentration bei zwischen 1,5 g/l und 7 g/l in dem wässrigen Medium gehalten wird.

15. Verwendung gemäß einem der Ansprüche 11 bis 14, wobei die Acetatkonzentration bei etwa 2 g/l in dem wässrigen Medium gehalten wird, wobei "etwa" eine Variation innerhalb von 20 Prozent bezeichnet.

## Revendications

1. Méthode de production de propanol et/ou d'acide propionique, la méthode comprenant :
(b) la mise en contact d'au moins un propionogène avec du dioxyde de carbone et avec un milieu aqueux comprenant de l'éthanol et un acétate ;
dans laquelle l'acétate est maintenu à une concentration d'au moins 1 g/l dans le milieu aqueux pendant la période de réaction.

2. Méthode selon la revendication 1, dans laquelle l'éthanol et l'acétate sont produits de manière exogène.

3. Méthode selon la revendication 1 ou bien 2, dans laquelle le propionogène est choisi dans le groupe constitué par *Clostridium neopropionicum, Clostridium propionicum, Pelobacter propionicus, Desulfobulbus propionicus, Syntrophobacter wolinii, Syntrophobacter pfennigii, Syntrophobacter fumaroxidans, Syntrophobacter sulfatireducens, Smithella propionica, Desulfotomaculum thermobenzoicum,* sous-espèce *thermosyntrophicum, Pelotomaculum thermopropionicum,* et *Pelotomaculum schinkii.*

4. Méthode selon l'une quelconque des revendications précédentes, dans laquelle le propionogène est *Clostridium neopropionicum.*

5. Méthode selon l'une quelconque des revendications précédentes, dans laquelle la concentration en acétate est maintenue entre 1 g/l et 10 g/l dans le milieu aqueux.

6. Méthode selon l'une quelconque des revendications précédentes, dans laquelle la concentration en acétate est maintenue entre 1,5 g/l et 7 g/l dans le milieu aqueux.

7. Méthode selon l'une quelconque des revendications précédentes, dans laquelle la concentration en acétate est maintenue à environ 2 g/l dans le milieu aqueux, où par « environ » on entend une variation dans les limites de 20%.

8. Méthode selon l'une quelconque des revendications précédentes, la méthode comprenant une étape (a) de production d'éthanol et d'acétate, où l'étape (a) comprend :
(a) la mise en contact d'au moins une bactérie acétogène avec une source de carbone, où la source de carbone comprend du dioxyde de carbone et/ou du monoxyde de carbone.

9. Méthode selon la revendication 8, dans laquelle la bactérie acétogène est choisie dans le groupe constitué par *Clostridium autoethanogenum* DSMZ 19630, *Clostridium ragsdalei* ATCC n° BAA-622, *Clostridium autoethanogenum, Moorella* sp. HUC22-1, *Moorella thermoaceticum, Moorella thermoautotrophica, Ruminococcus productus, Acetoanaerobium, Oxobacter pfennigii, Methanosarcina barkeri, Methanosarcina acetivorans, Carboxydothermus, Desulfotomaculum kuznetsovii, Pyrococcus, Peptostreptococcus, Butyribacterium methylotrophicum* ATCC 33266, *Clostridium formicoaceticum, Clostridium butyricum, Lactobacillus delbrueckii, Propionibacterium acidipropionici, Proprionispera arboris, Anaerobiospirillum succiniciproducens, Bacteroides amylophilus, Bacteroides ruminicola, Thermoanaerobacter kivui, Acetobacterium woodii, Acetoanaerobium notera, Clostridium aceticum, Butyribacterium methylotrophicum, Moorella thermoacetica, Eubacterium limosum, Peptostreptococcus productus, Clostridium ljungdahlii, Clostridium* ATCC 29797 et *Clostridium carboxidivorans.*

10. Méthode selon la revendication 8 ou 9, dans laquelle les étapes (a) et (b) sont mises en œuvre dans un fermenteur unique.

11. Utilisation d'acétate afin d'augmenter la proportion d'éthanol converti en propanol et/ou en acide propionique par un propionogène dans un milieu aqueux, où l'acétate dans le milieu aqueux est maintenu à une concentration d'au moins 1 g/l pendant la période de réaction, où le propionogène est mis en contact avec du dioxyde de carbone et avec le milieu aqueux comprenant l'éthanol et l'acétate.

12. Utilisation selon la revendication 11, dans laquelle l'éthanol et l'acétate sont constitués d'éthanol et d'acétate produits de manière exogène.

13. Utilisation selon la revendication 11 ou bien 12, dans laquelle le propionogène est choisi dans le groupe constitué par *Clostridium neopropionicum, Clostridium propionicum, Pelobacter propionicus, Desulfobulbus propionicus, Syntrophobacter wolinii, Syntrophobacter pfennigii, Syntrophobacter fumaroxidans, Syntrophobacter sulfatireducens, Smithella propionica, Desulfotomaculum thermobenzoicum,* sous-espèce *thermosyntrophicum, Pelotomaculum thermopropionicum,* et *Pelotomaculum schinkii.*

14. Utilisation selon l'une quelconque des revendications 11 à 13, dans laquelle la concentration en acétate est maintenue entre 1,5 g/l et 7 g/l dans le milieu aqueux.

15. Utilisation selon l'une quelconque des revendications 11 à 14, dans laquelle la concentration en acétate est maintenue à environ 2 g/l dans le milieu aqueux, où par « environ » on entend une variation dans les limites de 20%.
